(19) Europäisches Patentamt
European Patent Office
Office européen des brevets

(11) Veröffentlichungsnummer: **0 017 058**
**B1**

(12) **EUROPÄISCHE PATENTSCHRIFT**

(45) Veröffentlichungstag der Patentschrift: **10.03.82**

(51) Int. Cl.³: **C 07 D 209/48**

(21) Anmeldenummer: **80101367.3**

(22) Anmeldetag: **17.03.80**

(54) **Verfahren zur kontinuierlichen Herstellung von Phthalimid.**

(30) Priorität: **22.03.79 DE 2911245**

(43) Veröffentlichungstag der Anmeldung: **15.10.80 Patentblatt 80/21**

(45) Bekanntmachung des Hinweises auf die Patenterteilung: **10.03.82 Patentblatt 82/10**

(84) Benannte Vertragsstaaten: **BE CH DE FR GB**

(56) Entgegenhaltungen:
**AT-B-300 770**
**DE-A-2 334 379**
**DE-A-2 334 916**
**US-A-2 566 992**

(73) Patentinhaber: **BASF Aktiengesellschaft, Carl-Bosch-Strasse 38, D-6700 Ludwigshafen (DE)**

(72) Erfinder: **Kilpper, Gerhard, Dr., Waldstrasse 3, D-6719 Battenberg (DE)**
Erfinder: **Grimmer, Johannes, Duererstrasse 12, D-6700 Ludwigshafen (DE)**

**EP 0 017 058 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur kontinuierlichen Herstellung von Phthalimid

Die Erfindung betrifft ein Verfahren zur kontinuierlichen Herstellung von Phthalimid durch Umsetzung von Phthalsäureanhydrid mit Ammoniak in ungefähr stöchiometrischem Verhältnis in einer Mischvorrichtung und anschließend in einem Reaktionsrohr bei bestimmter Strömungsgeschwindigkeit und Temperatur.

Es ist aus der US-Patentschrift 1 966 068 bekannt, diskontinuierlich durch Einleiten von Ammoniak in eine Schmelze von Phthalsäureanhydrid und Sublimation des gebildeeten Endstoffs Phthalimid herzustellen; die Verwendung eines hohen Überschusses an Ammoniak, gerade auch im Falle von rohem Phthalsäureanhydrid als Ausgangsstoff, wird empfohlen. Weitere diskontinuierliche Ausführungsformen der Umsetzung beschreiben die österreichische Patentschrift 85 380 und die US-Patentschrift 2 668 326. Alle diese Verfahren haben den Nachteil des diskontinuierlichen Betriebs. Die Reaktionstemperatur kann nur langsam gesteigert werden, da andernfalls zusammen mit dem Phthalimid erhebliche Mengen an unumgesetztem Phthalsäureanhydrid, z. B. insgesamt 5 bis 50 Gewichtsprozent des gesamten Ausgangsanhydrids, sublimieren. Um einen möglichst vollständigen Umsatz zu erzielen, benötigen die Verfahren hohe Überschüsse an Ammoniak, z. B. die 4- bis 8fache stöchiometrische Menge Ammoniak, bezogen auf Phthalsäureanhydrid. Bei der Reaktion entsteht zunächst pro Mol Ammoniak ein Mol Wasserdampf; der Wasserdampf schleppt während der ersten Phase der Umsetzung reines Phthalsäureanhydrid im Abgas mit. Danach wird in immer stärkerem Maße je nach dem Grad der Umsetzung auch Phthalimid abgeführt. Phthalsäureanhydrid und das während der Reaktion gebildete Monoammoniumphthalat können sich bei höherem Umsatz und höherer Konzentration an Nebenstoffen im Abgas an den Anlagewänden ablagern und Verstopfungen von Rohren und Ventilen verursachen.

Es ist aus der US-Patentschrift 2 668 326 bekannt, daß man diskontinuierlich Phthalsäureanhydrid und Ammoniak umsetzt und die Abgase der Umsetzung mit Wasser oder einem hochsiedenden Kohlenwasserstoff als Lösungsmittel wäscht. Die Waschflüssigkeit wird vorteilhaft im Kreislauf geführt, wobei sich das suspendierte Phthalimid anreichert. Diese Suspension wird ebenfalls zum Abkühlen und Abschrecken des heißen Reaktionsgemisches verwendet.

In der deutschen Offenlegungsschrift 2 056 891 wird ein kontinuierliches Verfahren mit beiden Ausgangsstoffen im Gleichstrom beschrieben. Eine Schmelze von Phthalsäureanhydrid und überschüssigem Ammoniak wird in ein auf 200 bis 300°C erhitztes, senkrechtes Reaktionsrohr von oben eingeleitet und das sich biloende Phthalimid unten abgezogen. Das Reaktionsgemisch tritt in eine Sublimationskammer ein, wo durch Abkühlung Phthalimid niedergeschlagen wird, die nicht kondensierten Anteile an Phthalimid, Phthalsäureanhydrid und Nebenstoffen sowie Ammoniak und Wasserdampf werden über eine Gasschikane abgeleitet. Es ist ein erfindungswesentliches Merkmal des Verfahrens, daß Ammoniak im Überschuß von mindestens 20 Prozent, vorzugsweise in einem Überschuß von 25 bis 35 Gewichtsprozent, verwendet wird; die Raum-Zeit-Ausbeute errechnet sich zu 0,98 Teilen pro Stunde und Liter Reaktorraum.

In allen diesen Fällen befinden sich im Abgas noch Anteile an Phthalsäureanhydrid, die mindestens über 5, häufig über 20 Gewichtsprozent des Abgases betragen, was gerade im großtechnischen Maßstab unbefriedigend ist. Bei dem kontinuierlichen Verfahren sind im allgemeinen im Abgas neben Wasserdampf 5 bis 35 Gewichtsprozent Phthalsäureanhydrid, 20 bis 40 Gewichtsprozent Phthalimid, 3 bis 10 Gewichtsprozent Ammoniak, bezogen auf die Gesamtmenge Abgas, enthalten. Bei diesen bekannten Verfahren ergeben sich je nach Reaktionsbedingungen, Temperatur und Zusammensetzung des Reaktionsgemisches zum Teil erhebliche Verluste an Endstoff.

Bei dem in der deutschen Offenlegungsschrift 2 056 891 beschriebenen Verfahren wird versucht, den Verlust dadurch auf ein Minimum zu beschränken, daß die Abgase durch eine Vorlage mit Gasschikanen geleitet werden, die durch Umlenken des Abgasstromes bei gleichzeitigem Kühlen der Vorlage einen Teil des mitgeführten Phthalsäureanhydrid- und Phthalimiddampfes abscheiden. Nach dem im US-Patent 2 668 326 beschriebenen Verfahren werden die Phthalsäureanhydrid und Phthalimid enthaltenden Dämpfe durch einen mit Wasser betriebenen Wäscher geschickt, in dem der Wasserdampf kondensiert und die organischen Verbindungen auskristallisieren. Die entstehende Maische wird als Flüssigkeit zum Abschrecken (Quench) für die heiße Phthalimidschmelze benutzt. Nachteilig sind die umständlichen Operationen der Abtrennung des festen Phthalimids von der wäßrigen Phase mittels Filtration oder Zentrifugieren und Trocknung. In allen Fällen ist das Phthalimid mit Phthalsäureanhydrid verunreinigt und muß, beispielsweise durch fraktionierte Sublimation, gereinigt werden.

Es ist aus den deutschen Offenlegungsschriften 2 334 379 und 2 334 916 bekannt, daß man Phthalimid bei erhöhter Temperatur kontinuierlich durch Umsetzung von Ammoniak und Phthalsäureanhydrid im Gegenstrom und in einem Molverhältnis von 0,9 bis 1,1 zu 1 herstellt. Das bei der Umsetzung gebildete Abgas wird vorteilhaft mit wäßrigem Ammoniak gewaschen. Während im Abgas bei den vorgenannten diskontinuierlichen und kontinuierlichen Verfahren im großtechnischen Maßstab im allgemeinen 20 bis 40 Gewichtsprozent Phthalsäureimid, bezogen auf die Gewichtsmenge Abgas, verloren gehen, enthält das Abgas dieser beiden Verfahren ohne Wäsche mit wäßrigem

Ammoniak unterhalb 0,5 Gewichtsprozent Phthalimid und mit Wäsche unterhalb 0,001 Gewichtsprozent Phthalimid. Als Reaktoren kommen Rührkesselkaskaden, vorteilhaft mit 3 bis 6 hintereinander geschalteten Rührkesseln, oder Kolonnen, von denen ein Teil als Rührkessel wirkt und deren unterer Teil so ausgestattet ist, daß die Rekationspartner möglichst ohne Rückvermischung miteinander reagieren. Beispielsweise verwendet man Bodenkolonnen oder Blasensäulen, bei denen der untere Teil der Kolonne mit Füllkörpern gefüllt ist. Man kann auch einen Rührbehälter mit nachgeschalteter Gegenstromkolonne verwenden. Vorteilhaft verwendet man Bodenkolonnen. Zweckmäßig ist eine Temperatur von mindestens 150°C, insbesondere von 150 bis 210°C, am Kopf und von höchstens 270°C, insbesondere von 240 bis 265°C, am Boden der Kolonne, mit kontinuierlich steigender Temperatur von oben nach unten. Der Durchsatz von Ammoniak ist zweckmäßig von 90 bis 280, vorzugsweise von 160 bis 220 kg/h und qm Reaktorquerschnitt. Die beiden Ausgangsstoffe werden in einer Kolonne in vorgenannter Weise bei der Reaktionstemperatur im Gegenstrom miteinander umgesetzt. Am Boden der Kolonne wird das Reaktionsgemisch, im wesentlichen Phthalimid, als Schmelze abgezogen. Am Kopf der Kolonne tritt das Abgas aus.

Im allgemeinen enthält das Abgas beim vorgenannten Gegenstromverfahren 40 bis 60 Gewichtsprozent Wasserdampf, 0,3 bis 1 Gewichtsprozent Ammoniak, 35 bis 50 Gewichtsprozent Phthalsäureanhydrid, je nach Reaktionstemperatur 0 bis 6 Gewichtsprozent Phthalsäure, 0,3 bis 1 Gewichtsprozent Phthalimid, 0 bis 8 Gewichtsprozent Monoammoniumphthalat, 0 bis 10 Gewichtsprozent Diammoniumphthalat.

Die deutsche Offenlegungsschrift 2 334 916 lehrt, daß das Abgas der Umsetzung mit einer Schmelze, die mindestens 70 Gewichtsprozent Phthalimid enthält, bei einer Temperatur von mindestens 210°C gewaschen und die Schmelze dann der Umsetzung wieder zugeführt werden kann. Das Abgas enthält nach der Wäsche höchstens 1,5, meist unter 0,3 Gewichtsprozent Phthalsäureanhydrid und höchstens 70, meist unter 65 Gewichtsprozent Phthalimid. Im wesentlichen ist somit das Abgas von Nebenstoffen befreit und der Anteil an Phthalimid erhöht. Da man zweckmäßig das Abgas in wäßrigem Alkali, z. B. in 5- bis 20gewichtsprozentiger Natronlauge oder Kalilauge auffängt, wobei sich Phthalimid löst, können solche Lösungen z. B. direkt der Synthese von Anthranilsäure und Isatosäureanhydrid durch Umsetzung von phthalimidsaurem Alkali und Alkalihypochlorit als Ausgangsstoffe zugeführt werden. Schon ein geringer Anteil an Phthalsäureanhydrid würde andererseits sich ebenfalls in Alkali lösen und bei der folgenden Synthese unreine Endstoffe ergeben, was kostspielige, umständliche und an Endstoff verlustreiche Reinigungsoperationen verursacht.

Es wurde nun gefunden, daß man kontinuierlich Phthalimid durch Umsetzung von Phthalsäureanhydrid mit Ammoniak bei erhöhter Temperatur vorteilhaft herstellt, wenn man

a) Phthalsäureanhydrid in geschmolzenem Zustand mit Ammoniak in einem Molverhältnis von 1 bis 1,1 Mol Ammoniak je Mol Phthalsäureanhydrid in einer Mischvorrichtung bei einer Temperatur von 135 bis 300°C und einem Druck bis höchstens 50 bar mischt, umsetzt, dann
b) das so erhaltene Gemisch in einem Reaktionsrohr mit einer Strömungsgeschwindigkeit von oberhalb 0,01 Meter pro Sekunde bei 235 bis 300°C und einem Druck bis höchstens 50 bar weiter umsetzt und
c) aus dem abströmenden Umsetzungsgemisch in üblicher Weise Phthalimid abtrennt.

Die Umsetzung kann durch das folgende Schema wiedergegeben werden:

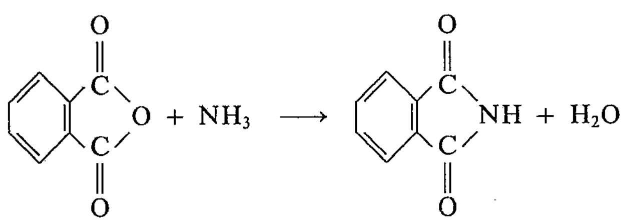

Im Vergleich zu den bekannten diskontinuierlichen Verfahren liefert das Verfahren nach der Erfindung auf einfacherem und wirtschaftlicherem Wege Phthalimid in besserer Raum-Zeit-Ausbeute und Reinheit. Mit Bezug auf das kontinuierliche, in der deutschen Offenlegungsschrift 2 056 891 beschriebene Verfahren ist das erfindungsgemäße Verfahren ebenfalls einfacher und wirtschaftlicher, da überraschend ein größerer Überschuß an Ammoniak nicht benötigt wird; auch ist die Ausbeute an reinerem Endstoff höher, da weniger Phthalsäureanhydrid, im allgemeinen unterhalb 0,5 Gewichtsprozent Phthalsäureanhydrid, praktisch kein Monoammoniumphthalat und kein Diammoniumphthalat als Nebenstoffe im gebildeten Endstoff vorhanden sind. Umständliche Reinigungsanlagen, eine Gasschikane und besondere Aufarbeitung des Endstoffs werden vermieden.

Der erfindungsgemäß hergestellte Endstoff ist somit von besonderer Bedeutung als Vorprodukt zur großtechnischen Herstellung von Anthranilsäure und Isatosäureanhydrid, wobei man vorteilhaft Phthalimid ohne Zwischenisolierung sofort in verdünnter Natronlauge auflöst und dem Hofmann-Abbau mit Bleichlauge zuführt. Gerade im Hinblick auf den Hofmann-Abbau ist es erforderlich, eine Phthalimidlösung zu verwenden, die praktisch kein freies Ammoniak enthält, da mit Bleichlauge explosive Chlor-Stickstoff-Verbindungen entstehen können; die erfindungsgemäße Herstellung ohne größeren Ammoniaküberschuß ist daher vorteilhaft. Der in flüssigem Zustand anfallende Endstoff kann ohne weitere Reinigung direkt zu Anthranilsäure oder Isatosäureanhydrid aufgearbeitet oder über eine Kühlwalze in fester Form isoliert werden.

Im Vergleich zu den in den deutschen Offenlegungsschriften 2 334 379 und 2 334 916 beschriebenen Verfahren ist das erfindungsgemäße Verfahren einfacher, wirtschaftlicher und liefert, wenn die Gesamtproduktion mit Aufarbeitung des Endstoffs in Betracht gezogen wird, eine höhere Raum-Zeit-Ausbeute. Es benötigt nur einen minimalen Aufwand an Meß- und Regeleinrichtungen, wodurch zusätzlich die Betriebssicherheit erhöht wird. Zieht man die Ausführungsbeispiele der bekannten kontinuierlichen Verfahren in Betracht, so erhält man im Falle der deutschen Offenlegungsschrift 2 056 891 einen Durchsatz von 1885 Kilogramm pro Stunde und Quadratmeter Reaktorquerschnitt, im Falle der deutschen Offenlegungsschrift 2 334 379 einen Durchsatz von 800 bis 3000 Kilogramm pro Stunde und Quadratmeter Reaktorquerschnitt, im Falle der deutschen Offenlegungsschrift 2 334 916 einen Durchsatz von 800 bis 3000 Kilogramm pro Stunde und Quadratmeter Reaktorquerschnitt. Bei dem erfindungsgemäßen Verfahren werden Durchsätze oberhalb von 10 000 Kilogramm pro Stunde und Quadratmeter Reaktorquerschnitt verwendet und ein rascherer Betrieb von Anlagen und Produktion erhalten. Alle diese vorteilhaften Ergebnisse sind im Hinblick auf den Stand der Technik überraschend.

Die Umsetzung wird mit einem Molverhältnis von 1 bis 1,1, vorzugsweise 1 bis 1,02, insbesondere 1 Mol Ammoniak zu 1 Mol Phthalsäureanhydrid, drucklos oder unter Druck, zweckmäßig bei einer Temperatur von 135 bis 300°C, vorzugsweise von 200 bis 280°C, insbesondere 240 bis 270°C, durchgeführt. Man kann reines Phthalsäureanhydrid oder im großtechnischen Maßstab einfacher und wirtschaftlicher technisches, rohes Phthalsäureanhydrid verwenden, z. B. mit Konzentrationen von 90 bis 95 Gewichtsprozent reinem Phthalsäureanhydrid, wie es z. B. bei der Herstellung durch katalytische Oxidation von Naphthalin oder o-Xylol mit Luft, anfällt. Ebenfalls kann Ammoniak rein oder mit inerten Gasen, z. B. Stickstoff oder Kohlendioxid, vermischt sein. Im allgemeinen wird Phthalsäureanhydrid als Schmelze mit einer Strömungsgeschwindigkeit von 0,005 bis 15, vorzugsweise von 0,1 bis 1 Meter/Sekunde und Ammoniak mit einer Geschwindigkeit von 0,1 bis 50, vorzugsweise von 1 bis 15 Meter/Sekunde in die Mischvorrichtung eingegeben. Zweckmäßig wird Phthalsäureanhydrid von einer Temperatur von 135 bis 300°C, vorzugsweise von 240 bis 270°C und Ammoniak von einer Temperatur 50 bis 300, vorzugsweise von 240 bis 280°C und einem Druck von 1 bis 50, vorzugsweise von 2 bis 10 bar verwendet. Über eine Regelvorrichtung kann Ammoniak an einer oder mehreren Stellen der Mischvorrichtung zugegeben werden.

In einer bevorzugten Ausführungsform des Verfahrensschrittes a) werden die Ausgangskomponenten in einer Mischvorrichtung intensiv durchmischt, vorzugsweise mit einer Durchwirbelung in Strahldüsen von Phthalsäureanhydrid und Ammoniak, die jeweils beim Eintritt in diese Strahldüsen die vorgenannten Strömungsgeschwindigkeiten besitzen. Andere Mischvorrichtungen werden zweckmäßig so eingestellt, daß die Durchmischungsgeschwindigkeit die der vorgenannten Strahldüsen entspricht. Die erfindungsgemäße Einstellung dieser Durchmischungsgeschwindigkeit kann durch einen Vergleichsversuch mit dem Durchmischungseffekt in Strahldüsen leicht festgelegt werden. Unter Verwendung vorgenannter Durchmischungsbedingungen können in einem breiten Maße bekannte Rührvorrichtungen verwendet werden: Injektoren, Kugeldüsen, Dralldüsen, Turbinenrührer, Mischdüsen, Lechler-Mischdüse, Spiralturbinen, Planetenrührwerke, Zentrifugalrührkreisel, rotierende Zerstäuber, Innenrohrmischer mit spiralförmig angeordneten, verstellbaren Umlenkblechen bzw. Füllkörpern, Impeller-Rührer; bevorzugt sind Impeller-Rührer, Strahldüsen (Treibstrahlmischer), Innenrohrmischer und Mischdüsen. Ebenfalls kommen Apparaturen und Anlagen, die eine intensive Durchmischung gestatten, wie Strömungsrohre, Rohrleitungs-T-Stücke, Mammutrührwerke, Homogenisierungsmaschinen, Kreiselmischer, Turbomischer, Durchlaufmischer, Strahlwäscher, Flüssigkeitsstrahl-Verdichter, Strahldüsen-Rohrreaktoren, in Frage. Die Verweilzeit in der Mischvorrichtung beträgt 0,001 bis 60, vorzugsweise 0,01 bis 10 Sekunden, der Druck zweckmäßig 1 bis 50, vorzugsweise 2 bis 10 bar, die Mischtemperatur, die sich durch Beheizung und/oder die direkte Wärmezufuhr der in die Vorrichtung eintretenden Ausgangsstoffe einstellt, beträgt zweckmäßig von 135 bis 300°C, vorzugsweise von 200 bis 280°C, insbesondere 240 bis 270°C. Der größere Teil, vorzugsweise 50 bis 80 Gewichtsprozent, des Phthalsäureanhydrids, wird zweckmäßig schon in der Stufe a), d. h. in der Mischvorrichtung, umgesetzt.

Aus der Mischungsvorrichtung gelangt das Reaktionsgemisch in den Reaktor (Stufe b), der aus einem beheizten Reaktionsrohr besteht, und von dort nach der Umsetzung in die Aufarbeitung. Das Reaktionsrohr kann in einer bevorzugten Ausführungsform Vorrichtungen enthalten, die die Durchmischung erhöhen, z. B. Füllkörper, Umlenkbleche oder Innenrohrmischer. Zweckmäßig stellt man durch einen engen Querschnitt des Reaktionsrohres und Verwendung entsprechender Transportpumpen eine hohe Strömungsgeschwindigkeit des Reaktionsgemisches in b) ein. Als Pumpen können z. B. Strahl-, Rotations-, Kreiskolben-, Wälzkolben-, Schraubenkolben-, Exzenter-, Flügel-, Kreisel-, Axial-, Propeller-pumpen verwendet werden. In einer bevorzugten Ausführungsform

des Verfahrens werden die Strömungsgeschwindigkeiten durch Querschnitt und Länge des Reaktionsrohres bestimmt. Zweckmäßig verwendet man Strömungsgeschwindigkeiten zwischen 0,01 und 50, insbesondere von 0,1 bis 15 Meter/Sekunde. Vorteilhaft sind z. B. Reaktorquerschnitte von 10 bis 200 000 $mm^2$, Rohrlängen von 0,1 bis 100, vorzugsweise 1 bis 30 Metern. Bei diesen Geschwindigkeiten wird in der Regel in einer Verweilzeit von 0,1 bis 600, vorzugsweise von 1 bis 30 Sekunden der Ausgangsstoff in der Stufe b) zu Ende umgesetzt. Die Reaktion wird in Stufe b) bei einer Temperatur von 235 bis 300° C, vorzugsweise von 240 bis 280° C, insbesondere von 245 bis 270° C, drucklos oder unter Druck, zweckmäßig 1 bis 50 bar, vorzugsweise 2 bis 10 bar, durchgeführt.

Am Ende des Reaktors wird das Reaktionsgemisch, im wesentlichen Phthalimid, als Schmelze abgezogen und auf z. B. einer Kühltrommel oder auf einem gekühlten Band abgekühlt und zweckmäßig in Schuppenform isoliert. In einer bevorzugten Ausführungsform des Verfahrens wird das aus dem Reaktor austretende Gemisch ohne Zwischenisolierung in verdünnter Natronlauge aufgenommen. Man erhält eine Lösung des Natriumsalzes der Phthalamidsäure, die sofort ohne weitere Reinigung zu Anthranilsäure oder Isatosäureanhydrid verarbeitet werden kann.

Das nach dem Verfahren der Erfindung herstellbare Phthalimid ist ein wertvoller Ausgangsstoff für die Herstellung von Farbstoffen, Schädlingsbekämpfungsmitteln und Pigmenten, insbesondere Kupferphthalocyaninen. Es ist Stabilisierungszusatz zu Flugzeugtreibstoffen. Bezüglich der Verwendung wird auf Ullmanns Encyklopädie der technischen Chemie, Band 13, Seite 735, verwiesen.

Die im folgenden Beispiel aufgeführten Teile bedeuten Gewichtsteile; sie verhalten sich zu Volumenteilen wie Kilogramm zu Liter.

## Beispiel 1

Aus einem beheizten Vorratsbehälter werden mit einer beheizten Kreiselpumpe 400 Teile/Stunde Phthalsäureanhydrid (0,21 Meter/Sekunde) mit einer Temperatur von 260° C einer Strahldüse zugeführt. An die Mischdüse ist ein mantelbeheiztes Reaktionsrohr (Reaktor) von 25 Millimeter Innendurchmesser in Form einer Rohrschlange angeschlossen. Die Gesamtlänge des Reaktors beträgt 30 Meter. 46 Teile Ammoniak (5,75 Meter/Sekunde) werden stündlich mit einer Temperatur von 260° C und einem Druck von 4 bar über eine mantelbeheizte Rohrleitung in die Strahldüse eingedüst. In der Strahldüse beträgt die Temperatur 260° C, der Druck 3,5 bar, die Verweilzeit 0,07 Sekunden. Die sofort beginnende, exotherm verlaufende Reaktion der Bildung des Phthalimids wird im nachfolgenden Reaktor zu Ende geführt. Im Reaktor beträgt die Temperatur 265° C, der Druck 3,5 bar, die Verweilzeit 2,05 Sekunden, die Strömungsgeschwindigkeit 14,7 Meter/Sekunde. Bereits nach 10 Meter Rohrstrecke zeigt die Reaktion schon einen Umsatz von über 99 Prozent. Man trennt den flüssigen Endstoff in einer Vorlage von dem gas/dampfförmigen Anteil des Reaktionsgemischs ab und erhält stündlich 316 Teile Phthalimid vom Schmelzpunkt 233,4° C neben 0,4 Prozent Phthalsäureanhydrid. Das die Vorlage verlassende Abgas (Wasserdampf, dampfförmiges Phthalimid, 0,4 Gewichtsprozent Ammoniak und 0,4 Gewichtsprozent Phthalsäureanhydrid) wird im Wasser aufgenommen und das ausgefallene Phthalimid abgetrennt. Man erhält stündlich 79 Teile Phthalimid mit einer Reinheit von 99,6 Gewichtsprozent neben 0,4 Gewichtsprozent Phthalsäureanhydrid und insgesamt 395 Teile/Stunde Phthalimid, entsprechend 99,4% der Theorie. Die Raum-Zeit-Ausbeute beträgt 27 Teile pro Stunde und Liter Reaktor. Der Durchsatz beträgt 806 000 Teile pro Stunde und Quadratmeter Reaktorquerschnitt. Das Phthalimid kann als solches direkt weiterverarbeitet werden. Der Betrieb kann ohne Verstopfung der Leitungen kontinuierlich durchgeführt werden.

## Beispiel 2

715 Teile Phthalsäureanhydrid (0,37 Meter/Sekunde) werden analog Beispiel 1 mit einer Temperatur von 260° C stündlich einer Strahldüse zugeführt, an die ein mantelbeheiztes Reaktionsrohr von 100 mm Innendurchmesser angeschlossen ist. Das Rohr ist auf einer Länge von 8 Meter mit Füllkörpern gefüllt. Die Gesamtlänge des Reaktors beträgt 10 m. 82,5 Teile Ammoniak (7,83 Meter/Sekunde) werden stündlich mit einer Temperatur von 260° C und einem Druck von 6 bar in die Strahldüse eingedüst. In der Strahldüse beträgt die Temperatur 260° C, der Druck 4,95 bar, die Verweilzeit 0,05 Sekunden. Im Reaktor beträgt die Temperatur 265° C, der Druck 4,95 bar, die Verweilzeit 7,8 Sekunden, die Strömungsgeschwindigkeit 1,27 Meter pro Sekunde. Analog Beispiel 1 erhält man stündlich 567 Teile Phthalimid (Fp 233,5) mit einer Reinheit von 99,8 Prozent neben 0,2 Prozent Phthalsäureanhydrid. Das den Behälter verlassende Abgas besteht aus Wasserdampf, dampfförmigem Phthalimid, 0,2 Gewichtsprozent Ammoniak und 0,2 Gewichtsprozent Phthalsäureanhydrid. Man erhält aus ihm stündlich 142 Teile Phthalimid mit einer Reinheit von 99,8 Prozent neben 0,2 Prozent Phthalsäureanhydrid. Die Gesamtausbeute beträgt 709 Teile Phthalimid pro Stunde, entsprechend 99,8% der Theorie. Die Raum-Zeit-Ausbeute beträgt 9 Teile pro Stunde und Liter Reaktor. Der Durchsatz beträgt 90 000 Teile pro Stunde und Quadratmeter Reaktorquerschnitt.

Beispiel 3

Das durch Mischen von 715 Teilen Phthalsäureanhydrid mit 82,5 Teilen Ammoniak gemäß Beispiel 2 erhaltene und aus dem Reaktor austretende Reaktionsgemisch wird in 2500 Teilen 25gewichtsprozentiger Natronlauge und 5700 Teilen Wasser unter Zusatz von 22 Teilen 30gewichtsprozentiger, wäßriger Lösung des Natriumsalzes der Sulfaminsäure gelöst und mit 2525 Teilen wäßriger Natriumhypochloritlösung (13,8 Gewichtsprozent aktives Chlor, 348 Teile Natriumhypochlorit) kontinuierlich umgesetzt. Man erhält stündlich 640 Teile Anthranilsäure (97% der Theorie) Fp 146,1° C, mit einer Reinheit von 99,9 Prozent.

**Patentanspruch**

Verfahren zur kontinuierlichen Herstellung von Phthalimid durch Umsetzung von Phthalsäureanhydrid mit Ammoniak bei erhöhter Temperatur, dadurch gekennzeichnet, daß man

a) Phthalsäureanhydrid in geschmolzenem Zustand mit Ammoniak in einem Molverhältnis von 1 bis 1,1 Mol Ammoniak je Mol Phthalsäureanhydrid in einer Mischvorrichtung bei einer Temperatur von 135 bis 300° C und einem Druck bis höchstens 50 bar mischt, umsetzt, dann
b) das so erhaltene Gemisch in einem Reaktionsrohr mit einer Strömungsgeschwindigkeit von oberhalb 0,01 Meter pro Sekunde bei 235 bis 300° C und einem Druck bis höchstens 50 bar weiter umsetzt und
c) aus dem abströmenden Umsetzungsgemisch in üblicher Weise Phthalimid abtrennt.

**Claim**

A process for the continuous preparation of phthalimide by reacting phthalic anhydride with ammonia at an elevated temperature, characterized in that

a) phthalic anhydride in the molten state is mixed, and reacted, with ammonia, using a ratio of from 1 to 1.1 moles of the latter per mole of the former, in a mixing apparatus at from 135 to 300° C under a pressure of at most 50 bar, thereafter
b) the mixture thus obtained is further reacted in a reaction tube at a flow velocity grater than 0.01 meter per second, at from 235 to 300° C under a pressure of at most 50 bar, and
c) phthalimide is isolated in a conventional manner from the reaction mixture which issues.

**Revendication**

Procédé pour la préparation en continu de phtalimide par réaction d'anhydride phtalique avec l'ammoniac à température élevée, caractérisé en ce que:

a) on mélange et on fait réagir de l'anhydride phtalique à l'état fondu avec de l'ammoniac dans un rapport molaire de 1 à 1,1 mol d'ammoniac par mol d'anhydride phtalique, dans un dispositif mélangeur, à une température de 135 à 300° C et sous une pression pouvant atteindre au maximum 50 bar, puis
b) on poursuit la réaction du mélange ainsi obtenu dans un tube de réaction à une vitesse d'écoulement de plus de 0,01 m/s à une température de 235 à 300° C et sous une pression pouvant atteindre au maximum 50 bar, et
c) on sépare de façon usuelle le phtalimide du mélange réactionnel qui s'écoule.